**Europäisches Patentamt**

(19) **European Patent Office**

**Office européen des brevets**

(11) **EP 0 537 243 B1**

(12) ## EUROPEAN PATENT SPECIFICATION

(45) Date of publication and mention
of the grant of the patent:
**11.06.1997 Bulletin 1997/24**

(21) Application number: **91912449.5**

(22) Date of filing: **04.07.1991**

(51) Int. Cl.$^6$: **A61B 5/00**

(86) International application number:
**PCT/GB91/01090**

(87) International publication number:
**WO 92/00699 (23.01.1992 Gazette 1992/03)**

(54) **MECONIUM MONITORING SYSTEM**

ÜBERWACHUNGSSYSTEM FÜR MEKONIUM

SYSTEME DE SURVEILLANCE DE MECONIUM

(84) Designated Contracting States:
**DE FR GB IT**

(30) Priority: **04.07.1990 GB 9014786**

(43) Date of publication of application:
**21.04.1993 Bulletin 1993/16**

(73) Proprietor: **BRITISH TECHNOLOGY GROUP
LIMITED
London SE1 6BU (GB)**

(72) Inventors:
• **GENEVIER, Eric Serge Gilles
London NW10 5JG (GB)**
• **STEER, Philip James
Kingston upon Thames, Surrey KT2 7BJ (GB)**

• **DANIELIAN, Peter James
London SW2 5BM (GB)**
• **RANDALL, Nigel John
London NW6 6TS (GB)**
• **SMITH, Robin Wyncliffe,
Blackett Laboratory
Prince Consort Road, London SW7 2BZ (GB)**

(74) Representative: **Bird, Christopher John et al
British Technology Group
Patents Department
101 Newington Causeway
London SE1 6BU (GB)**

(56) References cited:
**EP-A- 0 063 431        FR-A- 2 608 909
GB-A- 2 195 897**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

## Description

This application relates to a system for monitoring the quality of amniotic fluid during labour.

In recent years, it has become accepted that there is a need for more information concerning the condition within the uterus just prior to and during labour in order to predict and avoid birth complications. U.K. Patent Application No. 87 23605 (Publication No. 2195897) describes an intrauterine probe which enables certain conditions, especially the fetal heart rate and the intrauterine pressure to be continuously monitored during labour. Any monitoring system should desirably be non-invasive to the fetus and be capable of a continuous collection of data concerning the condition of interest.

During intrauterine life, the human fetus collects within its bowel a collection of debris known as meconium. Passage of meconium in utero occurs in about 10% of babies overall, probably as part of a sympathetic 'fright, flight or fight' reaction. Because regular uterine contractions interfere with maternal blood flow within the placenta, the mean oxygen tension in fetal blood during labour drops from about 5 to 3kPa. This is thought to be the major stimulus causing the fetus to pass meconium in utero; it occurs with increasing frequency as gestation advances, reaching almost one third of all fetuses by 42 weeks gestation.

In 90% of fetuses who pass meconium into the amniotic fluid there are no harmful effects. However, in about 10% of cases the fetus gasps, inhaling the sticky, particulate meconium into the upper respiratory tract. Once the baby is born, this particulate matter produces partial airways obstruction, leading to inability to inflate alveoli in some areas, and hyperinflation in others. This disease is known as meconium aspiration syndrome (MAS).

Attempts have been made to prevent MAS by careful suctioning of the baby's pharynx immediately after the head is delivered; unfortunately such measures are largely ineffective.

FR-A-2608909 describes apparatus for monitoring amniotic fluid in utero which comprises a probe having a transparent distal end and being optically connected to a source of light and spectral analysis means. The probe does not, however, permit amniotic fluid to enter and does not distinguish from light reflected from the wall of uterus.

It appears that there is, as yet, no known effective prevention of this crippling and disabling condition. The main factor preventing progress is that the appearance of meconium into the amniotic fluid often remains undetected because the tight fit of the head in the pelvis does not allow amniotic fluid to drain out and become visible to the attending obstetrician.

There is therefore a need for a method of reliably monitoring the onset of meconium passage into the amniotic fluid and particularly one which can be carried out non-invasively to the fetus and continuously throughout labour.

According to one aspect of the present invention, there is provided a system for monitoring the content of amniotic fluid (AF) during labour which comprises a probe capable of insertion into a uterus during labour and being connected optically to a source of light and to spectral analysis means for analysing the spectral response of the AF to illumination, characterised in that an optical cell is supported in the probe and said cell is connected to the source of light and to the spectral analysis means and has an aperture for admission of AF, and wherein said cell is orientated in the probe in such a way that, in use, the AF is able to enter the cell and is illuminated by said source of light while avoiding any reflection of light from the wall of the uterus reaching the spectral analysis means.

According to a second aspect of the present invention there is provided an intrauterine probe which comprises a flexible elongated body having an optical cell housed therein, and an aperture in said cell for admission of amniotic fluid (AF), the cell being optically connected by fibre optic cable to the proximal end of the body and the cell being located and orientated in the body such that in use, amniotic fluid is able to pass through the cell and light passed down the cable from the proximal end is reflected back into the fibre optic cable by the AF while avoiding any reflection from the wall of the uterus entering the fibre optic cable.

Further features and advantages of the present invention will become apparent from the following detailed description and accompanying drawings, in which:-

Figure 1 is an absorption spectrum of three samples A, B and C of different amniotic fluids.
Figure 2 is an absorption spectrum of a blood-stained sample of amniotic fluid with expected base line.
Figure 3 is a perspective diagrammatic view of an intrauterine probe in accordance with the invention.
Figure 4 is an exploded view of an optical cell on an enlarged scale.
Figure 5 is a block diagram illustrating the signal processing equipment.

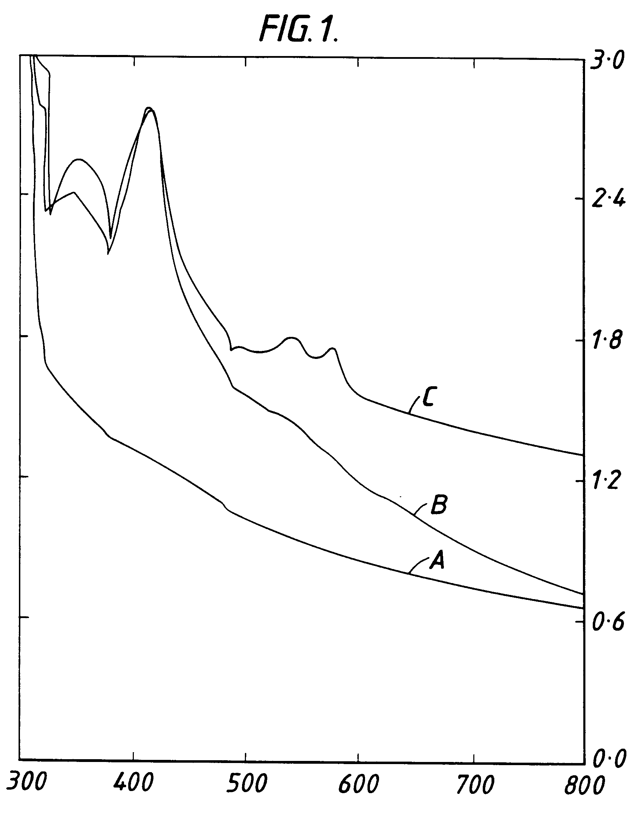

Two difficulties in making direct observations of the absorption spectrum of amniotic fluid are first to differentiate the spectra for meconium and blood, and secondly, to take into account the differing overall opacities between different samples of amniotic fluid. Typical spectra are shown in Figure 1, where A is the spectrum of a sample clear of meconium and blood, whereas B is the spectrum of a meconium stained sample showing a strong absorption peak in the range of 405 to 415nm, and C that of a blood stained sample. It can be seen that spectrum C has a major peak in the range of 405 to 415nm and two minor peaks in the range 535 to 545nm and 570 to 580nm.

The effect of blood on the absorption spectrum is important as it affects the whole spectrum in the range 405 to 415nm, where it adds a wide band peak in the same way as meconium does, the main difference between the two spectra being the two peaks seen at 540 and 575nm, and the fact that meconium absorbs poorly above about 650nm.

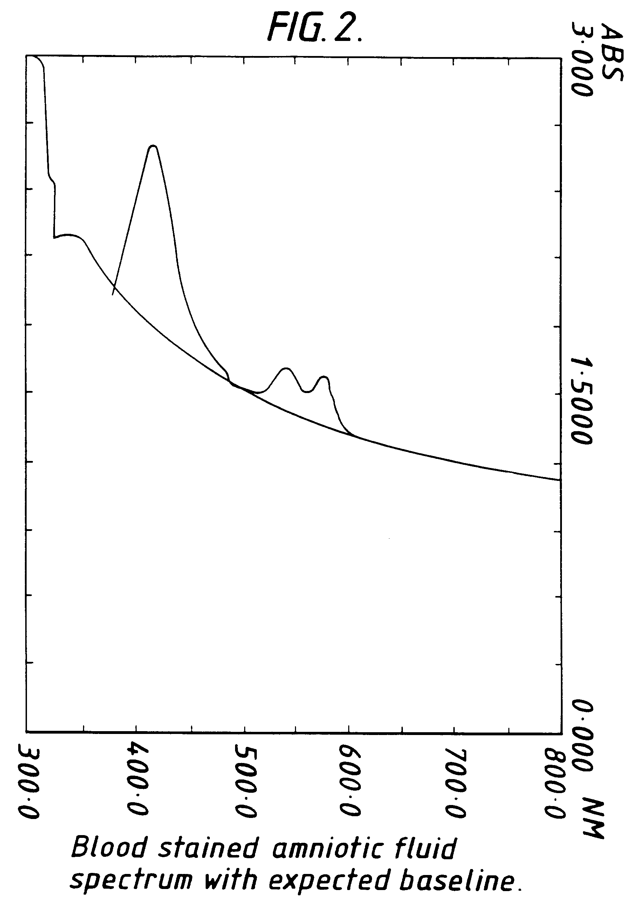

In order to account for the different opacities and distinguish between clear, meconium or blood-stained amniotic fluid, the system of the present invention involves a comparison of the spectral response at different wavelengths. One way in which this can be done is to measure the absorption spectrum of the amniotic fluid in the areas corresponding to the peaks produced by meconium and blood and at a third wavelength which is distant from the peaks characteristic of meconium and blood. In this way a base line value can be subtracted from the peak absorption values of the fluid under test. This procedure is illustrated graphically in Figure 2 which is a spectrum of a blood-stained sample of amniotic fluid containing meconium. It will be seen from Figure 2 that the point at 500nm can be taken as the base line. From these three absorptances we can derive the following variables:-

$$\delta(405) = K1 \cdot [\, A(405) - A(500) \,]$$

$$\delta(575) = K2 \cdot [\, A(500) - A(575) \,]$$

where :  A(405) = absorptance at 405nm
A(500) = absorptance at 500nm
A(575) = absorptance at 575nm

These variables are referred to hereinafter as the compensated absorptances. K1 and K2 are arbitrary constants to bring both values in the range 0 to 1.

For a sample clear of meconium and blood, $\delta(405)$ and $\delta(575)$ are both positive. As the level of meconium is increased, both $\delta(405)$ and $\delta(575)$ should increase but in the case where blood is also present, $\delta(575)$ does not increase significantly and often decreases to become negative, this is because the addition to the spectrum of a peak at 575nm reduces the difference A(500)-A(575).

Therefore, should blood appear in the amniotic fluid when meconium is already present, blood would immediately be detected, which is important as the appearance of blood in the amniotic fluid is a life-threatening condition requiring immediate attention.

While absorptance measurements can be used to discriminate amniotic fluid containing meconium or blood and clear fluid, a perfectly normal amniotic fluid may be extremely turbid because of the presence of vernix (a waxy substance which covers the fetus). In such circumstances, the vernix contamination makes it difficult to obtain meaningful absorptance measurements, except possibly in very thin films or after removal of a sample and centrifugation to clear the sample. The latter procedure is not usually possible where continuous monitoring is required.

A preferred procedure for analysing the spectral response of the amniotic fluid to illumination involves measurement of light back scattered (i.e. reflected) from a sample of the fluid. An amniotic fluid which is contaminated with vernix has a milky appearance and reflects light strongly over a wide waveband. As meconium increases in the amniotic fluid, it is also responsible for scattering light. Further, increased turbidity of the fluid will result in a reflected signal of increased strength.

It is, however, possible to distinguish increases in reflectance due to the presence of meconium, as opposed to vernix or other contaminants, by exploiting the optical absorptance characteristics of meconium as mentioned above. Because meconium absorbs light in the range of about 402 to 420nm, measurement of reflected values in this waveband compared with reflectance values at a wavelength where there is no significant absorption by meconium makes it possible to estimate the concentration of meconium. Observations have shown that light reflected from a sample of the fluid at 405 to 420nm increases with meconium concentration until a certain point (which is not necessarily the same from one sample to the next), where it starts to decrease due to the absorbing properties of meconium in that range. In contrast, reflectance values at, for example, 700nm continue to increase up to the maximum concentration measured (100g per litre of meconium). Blood can be differentiated in a similar way by measuring the reflectance at a third wavelength where blood absorbs light but meconium does not (540nm has been found to be suitable) and also at 700nm, where like meconium its absorptance is small. Although Figure 1 may suggest at first sight that light absorptance by blood at 700nm is significant, the spectrum at 700nm is, in fact, the overall attenuation of a light beam due to both absorption and scattering of light. According to the Beer Lambert Law, the intensity I of a monochromatic light beam of initial intensity $I_o$ passing through a medium of path length d and concentration c is:-

$$I = I_o e^{-ad}$$

where a is the attenuation coefficient which is plotted in Figures 1 and 2. The attenuation coefficient is, in fact, the sum of the absorption and scattering coefficients and is related to concentration of the substance under investigation in the following way:-

$$a = (a_1 + a_2)c$$

where $a_1$ is the absorption coefficient and $a_2$ is the scattering coefficient.

The scattering coefficient for blood is much larger than for meconium, but the absorption coefficients of both meconium and blood are insignificant at 700nm and can be ignored. Therefore, 700nm provides a suitable base line for concentration determinations when using reflectance measurements for both blood and meconium by logarithmic treatment of the reflectance measurements at 405 to 420nm, 540nm and 700 nm.

Thus, by measuring the intensity of the illuminating light (and its spectral content), the intensity of incident light at each wavelength of interest (e.g. 405-420, 540 & 700nm) can be calculated. Using these values and the values of reflected light at the same three wavelengths, the reflection due to the vernix content can be allowed for and compensated reflectance values calculated which are related to the content of meconium and blood in the amniotic fluid. From these compensated values, the concentration of meconium and blood can be calculated with a reasonable degree of accuracy.

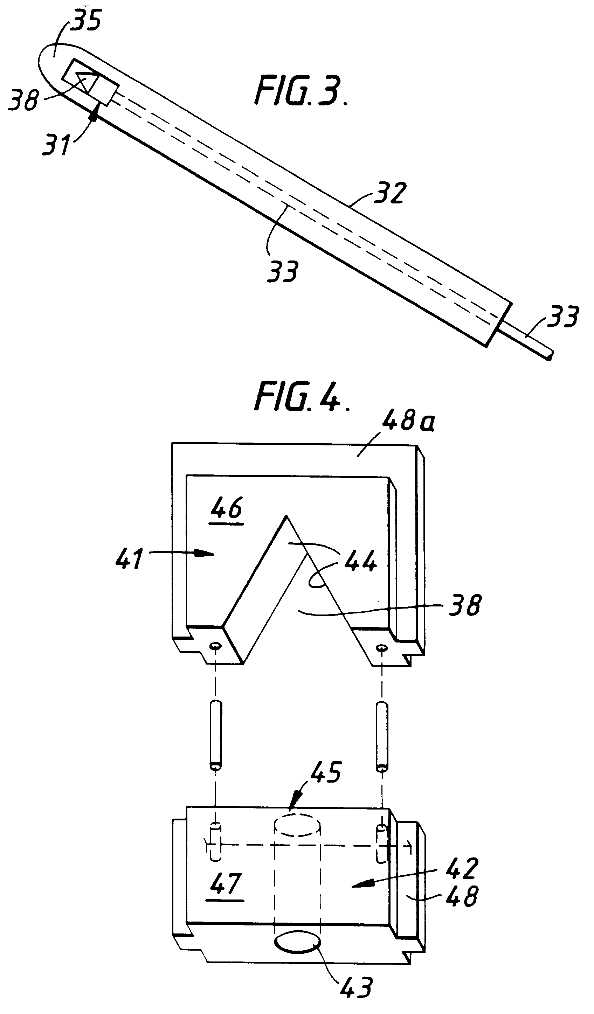

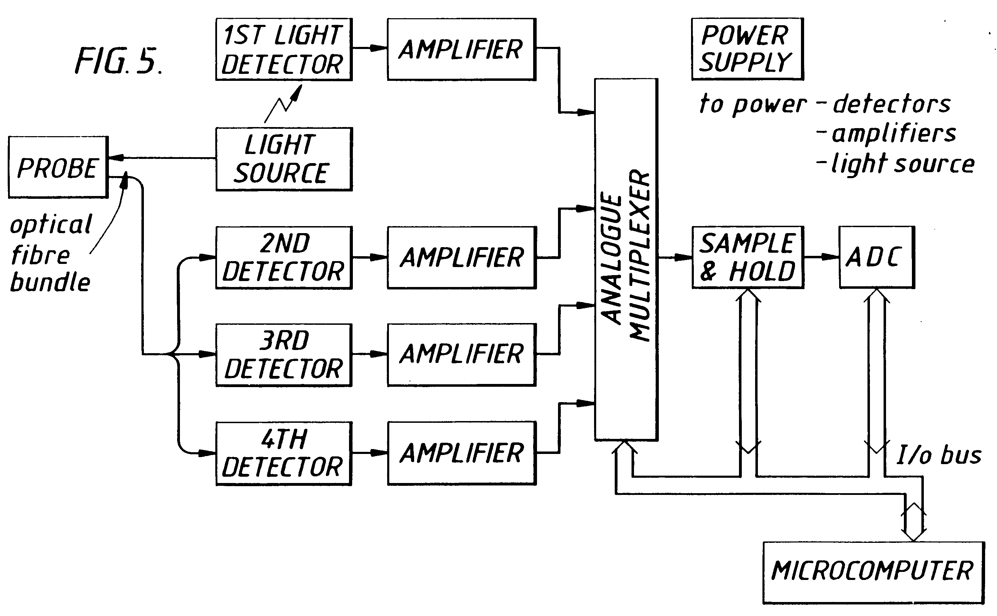

Optical measurements can be conveniently made in vivo, using a flexible intrauterine probe of the kind described in British patent application No. 8723605 (Publication No. 2195897). This is illustrated diagrammatically in Figure 3. As described in our above application, the probe is moulded from a suitable plastics material such as polyurethane and is stiff and resilient enough to enable it to be inserted by pushing into the uterus from the proximate end through the cervix and around the fetal head. The shape and dimensions and mechanical properties of the body of the probe are preferably as described in UK Patent Specification No. 2195897. Optical measurements are made using an optical cell which is encapsulated in a potting composition from which the probe is formed. The optical cell is preferably constructed in such a way that fluid can flow from one side of the probe through to the other by passing through passages in the cell. Thus referring to Figure 3, the optical cell 31 is encapsulated in the body 32 of the probe and the cell is optically connected to an optic fibre bundle 33, also encapsulated in the body 32. Bundle 33 emerges from the proximate end of the probe from which it is optically connected (indicated diagrammatically in Figure 5) to photodetectors.

The cell is shown in Figure 4 and is manufactured in two inter-engaging parts (41 & 42). Figure 4 shows the cell in an exploded view. Part 42 includes a bore 43 (typically about 3mms in diameter), for receiving the fibre optic bundle 33 (not shown in Figure 4). The fibre optic bundle comprises a large number of optic fibres which are located in bore 43 and preferably sealed in place with a suitable thermoplastic resin, e.g. an epoxy resin to prevent ingress of amniotic fluid by capillary action. Suitable optic fibre bundles are supplied by Fibre Data Limited, Unit 8, Pool Industrial Estate, Druids Road, Redruth, Cornwall TR15 3RH. The particular fibre bundle employed contained about 540 silica fibres and was randomly divided into two equal branches (known as a 'Y' shape bifurcated bundle), one branch serves to transmit light into the optical cell and the other collects the reflected light and conveys it to the spectrophotometer, comprising the photodetectors. When assembled, part 42 is secured to part 41 by the screws or studs indicated. Part 41 is shaped to provide conical or triangular-shaped counter-surfaces which prevent light which strikes such surfaces from being reflected back into the optic fibre-ends 45 of bore 43. Preferably, the internal faces 44 are coated with an anti-reflecting material or are roughened to minimise reflection.

When encapsulated in the material forming the body of the probe, faces 46 & 47 of parts 41 & 42 will be parallel with the flat surfaces 35 of the probe and flanges 48 & 48A of parts 41 & 42 will serve to lock the parts into the body of the probe. However, a passage 38 remains open through the probe and cell so that in use amniotic fluid may pass through the cell from one side of the probe to the other. Because the flat face 35 of the probe will in use lie against the wall of the uterus, light cannot be reflected from the uterus into the optic fibre bundle which is important since the wall of the uterus is red and would give a spurious signal.

Figure 5 shows a block diagram of the processing system. As illustrated in the block diagram, light from the source is fed to the probe via the optic fibre bundle and a first light detector is linked to the light source and measures its intensity. Reflected light is returned from the probe to second, third and fourth photodetectors (which may be photodiodes) and are tuned to detect the reflectance values at the three wavelengths of interest, i.e. 405-420nm, 540nm, and 700nm, respectively. After amplification, the signals are fed via an analogue multiplexer and an analogue-digital converter to a microcomputer.

The light source used to illuminate the sample passing the optical cell may be any suitable light source of sufficient intensity at the three desired wavelengths.

Preferably, the light source is a tungsten halogen filament bulb, although any light source emitting light at 415, 540 and 700nm would be suitable. The tungsten halogen lamp has the advantage that, while the total intensity fluctuates with temperature, the spectral content remains constant. Thus, by measuring overall intensity, the intensity delivered at each of the three selected wavelengths can easily be calculated.

The optical components and electronics circuitry can be housed together in a box adjacent to the bed and linked to probe. The output from this box may be taken to a central processing unit such as a microcomputer via a suitable interface. This arrangement enables the compensated reflectances to be calculated automatically and in real time, logged and displayed on a VDU or any suitable display in terms of corresponding concentrations of meconium and blood, or a hard copy record to be produced. Alternatively, a simpler read-out can be employed which merely gives an indication, when meconium and/or blood is detected at a level which suggests cause for alarm.

**Claims**

1. A system for monitoring the content of amniotic fluid (AF) during labour which comprises a probe (32) capable of insertion into a uterus during labour and being connected optically to a source of light and to spectral analysis means for analysing the spectral response of the AF to illumination, characterised in that an optical cell (31) is supported in the probe and said cell is connected to the source of light and to the spectral analysis means and has an aperture (38) for admission of AF, and wherein said cell is orientated in the probe in such a way that, in use, the AF is able to enter the cell and is illuminated by said source of light while avoiding any reflection of light from the wall of the uterus reaching the spectral analysis means.

2. A system according to claim 1 in which the means for analysing the spectral response of the AF to illumination comprises means for optically connecting the cell to photodetecting means whereby light back scattered by the AF in the cell is measured by the photodetecting means.

3. A system according to claim 1 or claim 2 in which the optical cell is connected to the photodetecting means and to the light source by fibre optic cable (33).

4. A system according to claim 3 in which the fibre optic cable comprises a bundle of intermingled fibres, some of said fibres being optically connected at the end remote from the cell with the light source and others with the means for analysing the response.

5. A system according to any one of the preceding claims in which the spectral response is analysed by determining the light reflected at first and second wavelengths at which meconium and blood, respectively, strongly absorb light and at a third wavelength at which meconium and blood do not absorb light significantly.

6. A system according to claim 5 in which the first wavelength is about 405 to 420 nm, the second wavelength is about 540 nm and the third wavelength is about 700 nm.

7. An intrauterine probe which comprises a flexible elongated body (32) having an optical cell (31) housed therein, and an aperture (38) in said cell for admission of amniotic fluid (AF), the cell being optically connected by fibre optic cable (33) to the proximal end of the body and the cell being located and orientated in the body such that in use, amniotic fluid is able to pass through the cell and light passed down the cable from the proximal end is reflected back into the fibre optic cable by the AF while avoiding any reflection from the wall of the uterus entering the fibre optic cable.

8. A probe according to claim 7 wherein the interior of the cell is provided with non-reflecting surfaces (44).

9. A probe according to claim 7 or 8 in which the fibre optic cable comprises a bundle of fibres, some of the fibres serving to transmit light and others to convey light reflected by the amniotic fluid to the proximal end for detection by photodetecting means, the fibres which transmit light and the fibres which receive reflected light being intermingled in the bundle.

**Patentansprüche**

1. System zur Überwachung des Gehalts von Fruchtwasser (FW) während der Wehen, das eine Sonde (32) umfaßt, die während der Wehen in einen Uterus eingeführt und optisch mit einer Lichtquelle und einer Spektralanalyseeinrichtung verbunden werden kann, zum Analysieren der spektralen Reaktion des FW auf Beleuchtung, dadurch gekennzeichnet, daß eine optische Zelle (31) in der Sonde abgestützt ist und die Zelle mit der Lichtquelle und der Spektralanalyseeinrichtung verbunden ist und eine Öffnung (38) zum Einlaß von FW aufweist, und wobei die Zelle in der Sonde auf solche Weise ausgerichtet ist, daß während des Gebrauchs das FW in die Zelle eintreten kann und durch die Lichtquelle beleuchtet wird, wobei jede Lichtreflektion von der Uteruswand, die die Spektralanalyseeinrichtung erreichen könnte, vermieden wird.

2. System nach Anspruch 1, wobei die Einrichtung zum Analysieren der spektralen Reaktion des FW auf Beleuchtung Einrichtungen zum optischen Verbinden der Zelle mit einem Photodetektor umfaßt, wobei Licht, das durch das FW in der Zelle zurückgestrahlt wird, durch den Photodetektor gemessen wird.

3. System nach Anspruch 1 oder 2, wobei die optische Zelle mit dem Photodetektor und der Lichtquelle durch Glasfaserkabel (33) verbunden ist.

4. System nach Anspruch 3, wobei das Glasfaserkabel ein Bündel Fasern umfaßt, wobei einige der Fasern am von der Zelle entfernten Ende mit der Lichtquelle und andere mit der Einrichtung zum Analysieren der Reaktion optisch verbunden sind.

5. System nach einem der vorhergehenden Ansprüche, wobei die spektrale Reaktion durch Bestimmen des Lichts, das mit einer ersten und einer zweiten Wellenlänge, bei denen Mekonium bzw. Blut Licht stark absorbieren und mit einer dritten Wellenlänge, bei der Mekonium und Blut Licht nicht wesentlich absorbieren, analysiert wird.

6. System nach Anspruch 5,

wobei die erste Wellenlänge bei ungefähr 405 bis 420 nm, die zweite Wellenlänge bei ungefähr 540 nm und die dritte Wellenlänge bei ungefähr 700 nm liegt.

7. Intrauterine Sonde, die einen flexiblen länglichen Körper (32) mit einer darin untergebrachten optischen Zelle (31) und einer in der Zelle befindlichen Öffnung (38) zum Einlaß von Fruchtwasser (FW) aufweist, wobei die Zelle durch ein Glasfaserkabel (33) mit dem proximalen Ende des Körpers optisch verbunden und die Zelle im Körper so angeordnet und ausgerichtet ist, daß beim Gebrauch Fruchtwasser durch die Zelle treten kann und Licht, das vom proximalen Ende entlang dem Kabel weitergeleitet wird, durch das FW zurück in das Glasfaserkabel reflektiert wird, wobei jede Reflektion von der Uteruswand, die in das Glasfaserkabel eintreten könnte, vermieden wird.

8. Sonde nach Anspruch 7,
   wobei das Innere der Zelle mit nicht-reflektierenden Oberflächen (44) versehen ist.

9. Sonde nach Anspruch 7 oder 8,
   wobei das Glasfaserkabel ein Bündel Fasern umfaßt, von denen einige dazu dienen, Licht zu übertragen und andere dazu, Licht, das durch das Fruchtwasser reflektiert wird, zum proximalen Ende zur Bestimmung durch den Photodetektor weiterzuleiten, wobei die Fasern, die Licht übertragen und die Fasern, die reflektiertes Licht empfangen im Bündel vermischt sind.

## Revendications

1. Système de contrôle du contenu du fluide amniotique (AF) pendant l'accouchement, qui comporte une sonde (32) capable d'être insérée dans un utérus pendant l'accouchement et étant reliée optiquement à une source de lumière et à des moyens d'analyse spectrale pour analyser la réponse spectrale du AF à un éclairage, caractérisé en ce qu'une cellule optique (31) est supportée dans la sonde et ladite cellule est reliée à la source de lumière et aux moyens d'analyse spectrale et a une ouverture (38) pour l'admission de AF, et dans lequel ladite cellule est orientée dans la sonde de telle manière que, en utilisation, le AF est capable d'entrer dans la cellule et est éclairé par ladite source de lumière, tout en évitant que toute réflexion d'une lumière à partir de la paroi de l'utérus n'atteigne les moyens d'analyse spectrale.

2. Système selon la revendication 1, dans lequel les moyens d'analyse de la réponse spectrale du AF à un éclairage comportent des moyens pour relier optiquement la cellule à des moyens de photodétection de sorte qu'une lumière rétrodiffusée par le AF dans la cellule est mesurée par les moyens de photodétection.

3. Système selon la revendication 1 ou 2, dans lequel la cellule optique est reliée aux moyens de photodétection et à la source de lumière par un câble à fibres optiques (33).

4. Système selon la revendication 3, dans lequel le câble à fibres optiques comporte un faisceau de fibres entremêlées, certaines desdites fibres étant reliées optiquement à l'extrémité éloignée de la cellule, la source de lumière et d'autres fibres étant reliées aux moyens d'analyse de la réponse.

5. Système selon l'une quelconque des revendications précédentes, dans lequel la réponse spectrale est analysée en déterminant la lumière réfléchie à des première et deuxième longueurs d'onde auxquelles le méconium et le sang, respectivement, absorbent fortement la lumière et à une troisième longueur d'onde à laquelle le méconium et le sang n'absorbent pas la lumière de manière significative.

6. Système selon la revendication 5, dans lequel la première longueur d'onde est d'environ 405 à 420 nm, la deuxième longueur d'onde est d'environ 540 nm et la troisième longueur d'onde est d'environ 700 nm.

7. Sonde intra-utérine qui comporte un corps allongé flexible (32) ayant une cellule optique (31) reçue dans celui-ci, et une ouverture (38) dans ladite cellule pour admission de fluide amniotique (AF), la cellule étant reliée optiquement par un câble à fibres optiques (33) à l'extrémité proximale du corps et la cellule étant située et orientée dans le corps de telle sorte qu'en utilisation, le fluide amniotique soit capable de passer à travers la cellule et que la lumière passée vers le bas du câble à partir de l'extrémité proximale soit réfléchie en retour dans le câble à fibres optiques par le AF tout en évitant toute réflexion par la paroi de l'utérus entourant le câble à fibres optiques.

8. Sonde selon la revendication 7, dans laquelle l'intérieur de la cellule est muni de surfaces non-réfléchissantes (44).

9. Sonde selon la revendication 7 ou 8, dans laquelle le câble à fibres optiques comporte un faisceau de fibres, certaines des fibres servant à transmettre la lumière et d'autres à amener la lumière réfléchie par le fluide amniotique à l'extrémité proximale pour détection par des moyens de photodétection, les fibres qui transmettent la lumière et les fibres qui reçoivent la lumière réfléchie étant entremêlées dans le faisceau.

## FIG.1.

# FIG. 2.

ABS
3.000

1.5000

0.000  NM

300.0   400.0   500.0   600.0   700.0   800.0

*Blood stained amniotic fluid
spectrum with expected baseline.*

8

FIG.3.

FIG.4.

FIG. 5.

PROBE

optical fibre bundle

1ST LIGHT DETECTOR → AMPLIFIER

LIGHT SOURCE

2ND DETECTOR → AMPLIFIER

3RD DETECTOR → AMPLIFIER

4TH DETECTOR → AMPLIFIER

ANALOGUE MULTIPLEXER

POWER SUPPLY

to power – detectors
– amplifiers
– light source

SAMPLE & HOLD → ADC

I/o bus

MICROCOMPUTER

EP 0 537 243 B1